(19) Europäisches Patentamt
European Patent Office
Office européen des brevets

(11) EP 0 933 026 A1

(12) **EUROPEAN PATENT APPLICATION**

(43) Date of publication:
**04.08.1999 Bulletin 1999/31**

(51) Int Cl.[6]: **A01N 31/04**

(21) Application number: **99300561.0**

(22) Date of filing: **26.01.1999**

(84) Designated Contracting States:
**AT BE CH CY DE DK ES FI FR GB GR IE IT LI LU MC NL PT SE**
Designated Extension States:
**AL LT LV MK RO SI**

(30) Priority: **30.01.1998 US 16362**

(71) Applicant: **American Cyanamid Company**
**Madison, New Jersey 07940-0874 (US)**

(72) Inventors:
- **Doscher, Mary Ehlers**
  **Trenton, New Jersey 08690 (US)**
- **Heaney, Kathleen**
  **Yardley, Pennsylvania 19067 (US)**
- **Schwinghammer, Kurt Allen**
  **Yardley, Pennsylvania 19067 (US)**

(74) Representative: **Connelly, Michael John et al**
**c/o Patent Department**
**Wyeth Laboratories**
**Huntercombe Lane South**
**Taplow**
**Maidenhead Berkshire SL6 0PH (GB)**

(54) **Compounds for protecting animals and humans against attack and infestation by arthropod parasites**

(57) The present invention relates to methods and compositions for treating animals and humans, and controlling, and preventing and protecting animals and humans from infestation and infection by arthropod parasites by administering or applying to the animals, humans or their environment a 1,4-diaryl-3-fluoro-2-butene compound having the structural formula

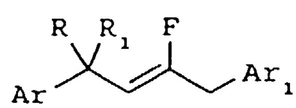

(I).

EP 0 933 026 A1

## Description

## BACKGROUND OF THE INVENTION

Arthropod and helminth parasites cause hundreds of millions of dollars in economic damage annually on a global basis. In particular, flies, fleas, lice, mosquitoes, gnats, mites, ticks and helminths cause tremendous losses to the livestock and companion animal sectors. Arthropod parasites also are a nuisance to humans and can vector disease causing organisms in humans and animals.

In spite of the commercial parasiticides available today, damage to humans, livestock and companion animals still occurs. Accordingly, there is ongoing research to create new and more effective parasiticides.

Certain 1,4-diaryl-3-fluoro-2-butene compounds which are useful for controlling whitefly are disclosed in WO 97/16067. And certain fluoroolefin compounds which are useful as soil insecticides are disclosed in WO 94/06741. However, neither WO 97/16067 nor WO 94/06741 describe any method for protecting animals and humans from attack and infestation by arthropod parasites.

U.S. 5,248,834 generically discloses certain fluoroolefin compounds which may be used to combat pests in several areas including medical areas. However, that patent does not specifically describe any fluoroolefin compounds. Furthermore, that patent does not provide a method for the preparation of any fluoroolefin compounds.

GB 2,288,803-A dislcoses certain fluoroolefin compounds which may be used to combat pests in several areas including medical areas. However, that patent application teaches away from medical applications because it discloses that the fluoroolefin compounds are particularly useful as soil insecticides.

It is, therefore, an object of the present invention to provide a method for treating animals and humans, and for controlling, and for preventing and protecting animals and humans from infestation and infection by, arthropod parasites.

It is also an object of the present invention to provide a composition for treating animals and humans, and for controlling, and for preventing and protecting animals and humans from infestation and infection by, arthropod parasites.

These and other objects of the present invention will become more apparent from the detailed description thereof set forth below.

## SUMMARY OF THE INVENTION

The present invention provides a method of treatment for controlling, preventing and protecting animals and humans from infestation and infection by arthropod parasites which comprises administering or applying to the animals, humans or their environment a parasiticidally effective amount of a 1,4-diaryl-3-fluoro-2-butene compound having the structural formula I

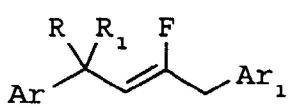

(I)

wherein

R is hydrogen or $C_1$-$C_4$alkyl, and

$R_1$ is $C_1$-$C_4$alkyl, $C_1$-$C_4$haloalkyl or cyclopropyl, or R and $R_1$ are taken together with the carbon atom to which they are attached to form a cyclopropyl group;

Ar is phenyl optionally substituted with up to three groups independently selected from halogen atoms, $C_1$-$C_4$alkyl groups, $C_1$-$C_4$haloalkyl groups, $C_1$-$C_4$alkoxy groups or $C_1$-$C_4$haloalkoxy groups, or 1- or 2-naphthyl optionally substituted with up to three groups independently selected from halogen atoms, $C_1$-$C_4$alkyl groups, $C_1$-$C_4$haloalkyl groups, $C_1$-$C_4$alkoxy groups or $C_1$-$C_4$haloalkoxy groups; and

$Ar_1$ is phenoxyphenyl optionally substituted with up to five groups independently selected from halogen atoms, $C_1$-$C_4$alkyl groups, $C_1$-$C_4$haloalkyl groups, $C_1$-$C_4$alkoxy groups or $C_1$-$C_4$haloalkoxy groups, phenyl optionally substituted with up to five groups independently selected from halogen atoms, $C_1$-$C_4$alkyl groups, $C_1$-$C_4$haloalkyl groups, $C_1$-$C_4$alkoxy groups or $C_1$-$C_4$haloalkoxy groups; biphenyl optionally substituted with up to five groups independently selected from halogen atoms, $C_1$-$C_4$alkyl groups, $C_1$-$C_4$haloalkyl groups, $C_1$-$C_4$alkoxy groups or

$C_1$-$C_4$haloalkoxy groups, benzylphenyl optionally substituted with up to five groups independently selected from halogen atoms, $C_1$-$C_4$alkyl groups, $C_1$-$C_4$haloalkyl groups, $C_1$-$C_4$alkoxy groups or $C_1$-$C_4$haloalkoxy groups, or benzoylphenyl optionally substituted with up to five groups independently selected from halogen atoms, $C_1$-$C_4$alkyl groups, $C_1$-$C_4$haloalkyl groups, $C_1$-$C_4$alkoxy groups or $C_1$-$C_4$haloalkoxy groups;

and the optical isomers thereof, and the configuration of the groups $ArCRR_1$- and -$CH_2Ar_1$ about the double bond is predominantly mutually trans.

This invention also provides compositions for the treatment of animals and humans by therapy, in particular, for controlling, preventing and protecting animals and humans from infestation and infection by arthropod parasites. The compositions of the present invention comprise an veterinarily or pharmaceutically acceptable carrier and an arthropod parasiticidally effective amount of a formula I compound. Advantageously, it has been found that the 1,4-diaryl-3-fluoro-2-butene compounds of this invention, and compositions containing them, are especially useful in the practice of this invention against infestation and infection by flies, fleas, lice, mosquitoes, gnats, mites and ticks.

## DETAILED DESCRIPTION OF THE INVENTION

The 1,4-diaryl-3-fluoro-2-butene compounds which are useful in the methods and compositions of this invention have the structural formula I

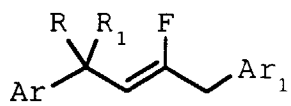

(I)

wherein R, $R_1$, Ar and $Ar_1$ are as described hereinabove for formula I.

Preferred formula I compounds for use in the methods and compositions of this invention are those wherein

- R is hydrogen and $R_1$ is isopropyl, trifluoromethyl or cyclopropyl, or R and $R_1$ are methyl, or R and $R_1$ are taken together with the carbon atom to which they are attached to form a cyclopropyl group;
- Ar is phenyl optionally substituted with up to three groups independently selected from halogen atoms, $C_1$-$C_4$alkyl groups, $C_1$-$C_4$haloalkyl groups, $C_1$-$C_4$alkoxy groups or $C_1$-$C_4$haloalkoxy groups; and
- $Ar_1$ is 3-phenoxyphenyl optionally substituted with up to five groups independently selected from halogen atoms, $C_1$-$C_4$alkyl groups, $C_1$-$C_4$haloalkyl groups, $C_1$-$C_4$alkoxy groups or $C_1$-$C_4$haloalkoxy groups, 3-biphenyl optionally substituted with up to five groups independently selected from halogen atoms, $C_1$-$C_4$alkyl groups, $C_1$-$C_4$haloalkyl groups, $C_1$-$C_4$alkoxy groups or $C_1$-$C_4$haloalkoxy groups, 3-benzylphenyl optionally substituted with up to five groups independently selected from halogen atoms, $C_1$-$C_4$alkyl groups, $C_1$-$C_4$haloalkyl groups, $C_1$-$C_4$alkoxy groups or $C_1$-$C_4$haloalkoxy groups, or 3-benzoylphenyl optionally substituted with up to five groups independently selected from halogen atoms, $C_1$-$C_4$alkyl groups, $C_1$-$C_4$haloalkyl groups, $C_1$-$C_4$alkoxy groups or $C_1$-$C_4$haloalkoxy groups.

More preferred formula I compounds are those wherein

- R is hydrogen and $R_1$ is isopropyl, trifluoromethyl or cyclopropyl, or R and $R_1$ are methyl, or R and $R_1$ are taken together with the carbon atom to which they are attached to form a cyclopropyl group;
- Ar is phenyl optionally substituted with up to three groups independently selected from halogen atoms, $C_1$-$C_4$alkyl groups, $C_1$-$C_4$haloalkyl groups, $C_1$-$C_4$alkoxy groups or $C_1$-$C_4$haloalkoxy groups; and
- $Ar_1$ is 3-phenoxyphenyl optionally substituted with up to five groups independently selected from halogen atoms, $C_1$-$C_4$alkyl groups, $C_1$-$C_4$haloalkyl groups, $C_1$-$C_4$alkoxy groups or $C_1$-$C_4$haloalkoxy groups.

Most preferred formula I compounds for use in the present invention are those wherein

- R is hydrogen and $R_1$ is isopropyl, trifluoromethyl or cyclopropyl, or R and $R_1$ are taken together with the carbon atom to which they are attached to form a cyclopropyl group;
- Ar is 4-chlorophenyl, 4-fluorophenyl, 4-(trifluoromethoxy)phenyl or 4-ethoxyphenyl; and
- $Ar_1$ is 4-fluoro-3-phenoxyphenyl, *m*-(*p*-fluorophenoxy)phenyl, 4-fluoro-3-(*p*-fluorophenoxy)phenyl or 3-phenoxyphenyl.

1,4-Diaryl-3-fluoro-2-butene compounds which are particularly useful for treating, controlling, preventing and protecting animals and humans from infestation and infection by arthropod parasites include
1-(*p*-chlorophenyl)-1-[2-fluoro-3-(4-fluoro-3-phenoxyphenyl)propenyl]cyclopropane, (Z)-;
2,5,5,5-tetrafluoro-4-(*p*-fluorophenyl)-1-(*m*-phenoxyphenyl)-2-pentene, (Z) -;
4-(*p*-chlorophenyl)-2-fluoro-1-(4-fluoro-3-phenoxyphenyl)-5-methyl-2-hexene, (Z) -;
1-[1-(*p*-chlorophenyl)-3-fluoro-4-(4-fluoro-3-phenoxyphenyl)-2-butenyl]cyclopropane, (Z) -;
1-{1-(*p*-chlorophenyl)-3-fluoro-4-[4-fluoro-3-(*p*-fluorophenoxy)phenyl]-2-butenyl}cyclopropane, (Z) -; and
1-[3-fluoro-4-(4-fluoro-3-phenoxyphenyl)-1-[*p*-(trifluoromethoxy)phenyl]-2-butenyl}cyclopropane, (Z)-, among others.

The methods and compositions of the present invention are useful against infestation and infection by arthropod parasites including, but not limited to, fleas, ticks, lice, mosquitoes, gnats, flies, biting flies, warble flies, muscoid flies, sheep ked, myiasitic fly larvae including those causing enteric myiasis and nasal bot, mites, chiggers and other biting, sucking and burrowing parasites of animals including, but not limited to, cattle, sheep, horses, deer, camels, swine, goats, ferrets, mink, rabbits, amphibians, reptiles, fish, birds, poultry, dogs and cats and humans. The methods and compositions of this invention are especially useful for treating, controlling, preventing and protecting animals and humans from infestation and infection by flies, fleas, lice, mosquitoes, gnats, mites and ticks.

The 1,4-diaryl-3-fluoro-2-butene compounds are effective against arthropod parasites of animals and humans from the Subclass Acari, including members of Orders Mesostigmata (e.g., fowl mites), Acariformes, including suborders Prostigmata (e.g., demodectic mites), Astigmata (e.g., psoroptic and sarcoptic mites) and Cryptostigmata (e.g., beetle mites) and Acarina, including members of Families Ixodoidea, Ixodidae (e.g., hard ticks) and Argasidae (e.g., soft ticks). The compounds are also effective against arthropod parasites of animals and humans of the Class Insecta, including members of the Orders Diptera (Simuliidae, Phlebotominae, Ceratopogonidae, Culcidae, Rhagionidae, Athericidae, Chloropidae, Sarcophagidae, Tabanidae, Muscidae, Hippoboscidae and Calliphoridae), Siphonaptera (including fleas of the genera *Ctenocephalides, Echidnophaga, Pulex,* and *Xenopsylla),* Malloghaga (including biting lice of the genera *Bovicola, Trichodectes* and *Damilina),* Anoplura (including sucking lice of the genera *Haematopinus, Linognathus* and *Solenopotes)* and Hemiptera. The compounds are useful during all life stages of the above mentioned parasites.

The methods and compositions of the present invention also may be useful against helminth parasites from the class Trematoda including members of the order Digenea (e.g., *Fasciola hepatica);* the class Cotyloda (e.g., *Diphyllobothrium* spp. and *Spirometra* spp.); the class Eucestoda (e.g., *Moniezia* spp., *Talnia* spp. and *Echinococcus* spp.); and the class Nematoda including members of the orders Rhabditida, Strongylida (e.g., *Haemonchus* spp., *Ostertagia* spp., *Cooperia* spp. and Ancyclostoma spp.), Oxyurida (e.g., Oxyuris spp.), Ascaridida (e.g., *Toxacara* spp.), Spirurida (e.g., *Dracunculus* spp., Thelazia spp., Onchocera spp. and Dirofilaria spp.), and Enoplida (e.g., *Trichuris* spp.).

The formula I compounds may be used in combination or conjunction with one or more other parasiticidal compounds including, but not limited to, anthelmintics, such as benzimidazoles, piperazine, levamisole, pyrantel, praziquantel and the like; endectocides such as avermectins, milbemycins and the like; ectoparasiticides such as arylpyrroles including chlorfenapyr, organophosphates, carbamates, gamabutyric acid inhibitors including fipronil, pyrethroids, spinosads, imidacloprid and the like; insect growth regulators such as pyriproxyfen, cyromazine and the like; and chitin synthase inhibitors such as benzoylureas including flufenoxuron.

The formula I compounds may also be used in combination or conjunction with one or more conventional synergists such as piperonyl butoxide, N-octyl bicycloheptene dicarboximide, dipropyl pyridine-2,5-dicarboxylate and 1,5a,6,9,9a,9b-hexahydro-4a(4H)-dibenzofurancarboxaldehyde to enhance efficacy, broaden spectrum and provide a convenient method for parasite control.

The formula I compounds may be applied topically to the skin, hide, fur, feathers or hair of the animal or human as a pour-on, spot-on, spray, dip, back-rubber, wettable powder, dust, emulsifiable concentrate, aqueous flowable, shampoo, paste, foam, cream, solution, suspension or gel. Those compositions generally contain about 0.1 ppm to 700,000 ppm of the active compound. In addition, the compound may be presented in the form of a solid matrix such as an ear tag, collar or medallion.

The formula I compounds may also be administered orally, intragastrically or intraruminally as a solution, paste, gel, tablet, bolus or drench or mixed in feed when prepared as a feed premix. Such administration is particularly useful for the control of enteric and somatic myiasis and to prevent development of the parasite in the feces and urine of treated animals and humans. In addition, the formula I compounds may be parenterally administered to the animals and humans.

The formula I compounds may also be applied to the environment in which the animals or humans inhabit as

a spray, powder, bait, solid matrix, solution, wettable powder, emulsifiable concentrate or fumigant. These environments may include, but are not limited to, homes, yards, barns, pens, kennels, poultry houses, feed lots, stables, stalls, offices, workplaces, food preparation facilities, dairies, fisheries, aviaries, zoos and the like.

The amount of active compound which is administered or applied to the animals, humans or their environment will vary depending upon the particular compound, target pest(s), application or administration method, and the particular host to be treated. Those skilled in the art can readily determine what a pesticidally effective amount is without undue experimentation.

The 1,4-diaryl-3-fluoro-2-butene compounds which are useful in the methods and compositions of this invention and methods for their preparation are described in GB 2,288,803-A; WO 94/06741; WO 97/16067; and co-pending U.S. Patent Application Serial Number 08/879,918 filed on June 19, 1997 published as EP 885,867A on 23 December 1998.

In order to facilitate a further understanding of the invention, the following examples are presented primarily for the purpose of illustrating more specific details thereof. The scope of the invention should not be deemed limited by the examples, but encompasses the entire subject matter defined in the claims.

## EXAMPLE 1

### Evaluation of test compounds against the adult cat flea, *Ctenocephalides felis*

This test is designed to evaluate the contact activity of test compounds against the adult cat flea. Test compounds are dissolved in 100% acetone to 300, 100, 10 and 1 ppm. 100 µL of each of these dilutions is added to the bottom of a 20 mL glass scintillation vial with a pipettor. The vial is allowed to dry under a fume hood for 15 minutes. The bottom of the vial measures 4.909 $cm^2$. The amount of test compound applied by each dilution equals 6.2, 2.0, 0.2 and 0.02 $\mu g/cm^2$, respectively. Newly emerged adult cat fleas are anesthetized with $CO_2$ to immobilize, and are counted into the scintillation vials with forceps. Five fleas are placed in each vial. These tests are done in replicates of two or three so that there are 10 to 15 fleas per rate/compound. Mortality readings are taken 4 and 24 hours after placing the fleas in the vials. Fleas which do not move when disturbed are considered dead. Percent mortality is then calculated for each treatment.

Data obtained are reported in Table I. Compounds employed in the evaluations of the present invention are given a compound number and identified by name. Data in Table I are reported by compound number.

## COMPOUNDS EVALUATED

| Compound Number | |
|---|---|
| 1 | 1-(*p*-Chlorophenyl)-1-[2-fluoro-3-(4-fluoro-3-phenoxyphenyl)propenyl]cyclopropane, (Z)- |
| 2 | 2,5,5,5-Tetrafluoro-4-(*p*-fluorophenyl)-1-(*m*-phenoxyphenyl)-2-pentene, (Z)- |
| 3 | 4-(*p*-Chlorophenyl)-2-fluoro-5-methyl-1-(*m*-phenoxyphenyl)-2-hexene, (Z)- |
| 4 | 4-(*p*-Chlorophenyl)-2-fluoro-1-(4-fluoro-3-phenoxy-phenyl)-5-methyl-2-hexene, (Z)- and (E)-, (8:1) |
| 5 | 2-Fluoro-4-(*p*-fluorophenyl)-5-methyl-1-(*m*-phenoxyphenyl)-2-hexene, (Z)- |
| 6 | 4-(*p*-Ethoxyphenyl)-2-fluoro-5-methyl-1-(*m*-phenoxyphenyl)-2-hexene, (Z)- |
| 7 | 1-[1-(*p*-Chlorophenyl)-3-fluoro-4-(4-fluoro-3-phenoxyphenyl)-2-butenyl]cyclopropane, (Z)- |
| 8 | 1-{1-(*p*-Chlorophenyl)-3-fluoro-4- [*m*-(*p*-fluorophenoxy) phenyl]-2-butenyl}cyclopropane, (Z)- |

(continued)

| Compound Number | |
|---|---|
| 9 | 1-{1-(*p*-Chlorophenyl)-3-fluoro-4-[4-fluoro-3-(*p*- fluorophenoxy)phenyl] -2-butenyl} cyclopropane, (Z)- |
| 10 | 1-{3-fluoro-4-(4-fluoro-3-phenoxyphenyl)-1-[*p*-(trifluoromethoxy)phenyl]-2-butenyl} cyclopropane, |

| Compound Number | |
|---|---|
| 1 | 1-(*p*-Chlorophenyl)-1-[2-fluoro-3-(4-fluoro-3-phenoxyphenyl)propenyl]cyclopropane, (Z)-(Z) - |

TABLE I

| Evaluation of Test Compounds against *Ctenocephalides felis* (cat flea) | | | |
|---|---|---|---|
| **Compound Number** | **Dose (μg/cm²)** | **4 Hour % Mortality** | **24 Hour % Mortality** |
| 1 | 6.20 | 100 | 100 |
| | 2.00 | 40 | 100 |
| | 0.20 | 40 | 60 |
| | 0.02 | 20 | 50 |
| 2 | 6.20 | 80 | 100 |
| | 2.00 | 30 | 100 |
| | 0.20 | 0 | 60 |
| 3 | 6.20 | 0 | 10 |
| | 2.00 | 0 | 0 |
| 4 | 6.20 | 0 | 90 |
| | 2.00 | 0 | 40 |
| | 0.20 | 0 | 0 |
| 5 | 6.20 | 0 | 20 |
| | 2.00 | 0 | 0 |
| 6 | 6.20 | 0 | 27 |
| | 2.00 | 0 | 20 |
| | 0.20 | 0 | 0 |
| 7 | 6.20 | 60 | 100 |
| | 2.00 | 40 | 80 |
| | 0.20 | 20 | 20 |
| | 0.02 | 0 | 0 |
| 8 | 6.20 | 0 | 60 |
| | 2.00 | 0 | 0 |
| 9 | 6.20 | 47 | 95 |

TABLE I (continued)

| Evaluation of Test Compounds against *Ctenocephalides felis* (cat flea) | | | |
|---|---|---|---|
| Compound Number | Dose (μg/cm²) | 4 Hour % Mortality | 24 Hour % Mortality |
| | 2.00 | 26 | 47 |
| | 0.20 | 0 | 21 |
| | 0.02 | 0 | 6 |
| 10 | 6.20 | 100 | 100 |
| | 2.00 | 60 | 100 |
| | 0.20 | 30 | 70 |
| | 0.02 | 0 | 10 |

## EXAMPLE 2

**Evaluation of test compounds aqainst the adult horn fly, *Haematobia irritans* (pyrethroid-susceptible)**

This test is conducted using 9 cm Whatman No. 1 filter papers which are placed in 100 x 20 mm disposable plastic petri dishes. Test doses are expressed as μg of test substance per $cm^2$ of filter paper.

1) Prepare solutions of test compound in acetone such that 800 or 1000 μL contains the amount necessary to obtain the desired dose rate.
2) Pipette 800 or 1000 μL of test solution onto filter paper.
3) Filter papers are dried under a fume hood.
4) When dry, filter papers are placed in the petri dish tops. Dishes are generally held about 24 hours before test organism is added.
5) Two- to five-day-old horn flies (pyrethroid-susceptible) which have had two or more blood meals are used in the assay. Flies are collected into a small vial from the holding cage. Vials are then chilled in a freezer for about 4 minutes to immobilize the flies. Immobilized flies are then distributed to petri dish bottoms, 12 to 25 flies per dish, then dishes are closed and inverted.
6) As soon as the flies become mobile (about 5 minutes), they are examined to detect any flies that are dead or injured. This number is subtracted from total number of flies in the dish.
7) Petri dishes are held at room temperature and examined at the desired time intervals which might be 10 minutes to 6 hours.
8) All dead flies are recorded at each examination time. A fly is considered dead if it cannot walk even though it may still be moving.
9) Each test has control dishes containing filter papers treated only with acetone.
10) Percent mortality at any time period is calculated using the following formula:

$$\frac{\text{Number of dead flies - number dead @ 5 min}}{\text{Total number of flies - number dead @ 5 min}} \times 100$$

Percent mortality is then corrected for control mortality using Abbot's formula.

Data for pyrethroid-susceptible horn flies are reported in Table II. The compounds evaluated are reported by compound number given in Example 1.

TABLE II

| Evaluation of Test Compounds against adult, Pyrethroid- susceptible horn fly *(Haematobia irritans)* | | |
|---|---|---|
| Compound Number | Dose (μg/cm²) | 6 Hour % Mortality |
| 1 | 20.00 | 100 |
| | 10.00 | 95 |
| | 5.00 | 53 |
| | 2.50 | 16 |

TABLE II (continued)

| Evaluation of Test Compounds against adult, Pyrethroid- susceptible horn fly *(Haematobia irritans)* | | |
|---|---|---|
| **Compound Number** | **Dose (μg/cm²)** | **6 Hour % Mortality** |
| | 1.25 | 3 |
| | 0.625 | 1 |
| | 0.3125 | 0 |
| 7 | 10.0 | 100 |
| | 5.00 | 98 |
| | 2.50 | 80 |
| | 1.25 | 15 |
| | 0.625 | 2 |
| | 0.3125 | 0 |

**EXAMPLE 3**

**Evaluation of test compounds against the adult house fly, *Musca domestica***

This test is designed to evaluate the activity of test compounds against the house fly. Test compounds are dissolved in 100% acetone to 300, 100 and 10 ppm. 100 μL of each of these dilutions is added to the bottom of a 20 mL glass scintillation vial with a pipettor. The vial is allowed to dry under a fume hood for 15 minutes. The vial is then turned on its side, and 250 μL of each dilution is applied to the sides of the vial. The vial is placed on a rolling machine and allowed to roll under the hood, coating the sides of the vial, until the acetone completely evaporates. The bottom of the vial measures 4.909 $cm^2$ and the sides measure 4.5 cm high x 8.8 cm around, so that the total coated area of the scintillation vial measures 44.51 $cm^2$. The amount of test compound applied by each dilution equals 2.36, 0.79, and 0.079 $\mu g/cm^2$, respectively. Included in all tests are two to three replications of untreated vials. Adult house flies which are five to seven days old are chilled in a freezer for five minutes to immobilize, and are placed on a sorting platform which provides a steady stream of $CO_2$ to keep the flies immobile. Five flies are counted into each scintillation vial with forceps. The top of the vial is plugged with a wad of sheet cotton sufficient to contain the flies. These tests are done in replicates of two to four so that there are 10 to 20 flies per rate/compound. Mortality readings are taken 1 and 4 hours after placing the flies in the vials. Flies which are unable to stand when disturbed are considered dead. Percent mortality is then calculated for each treatment.

Data obtained are reported in Table III. The compounds evaluated are reported by compound number given in Example 1.

TABLE III

| Evaluation of Test Compounds against *Musca domestica* (house fly) | | | |
|---|---|---|---|
| **Compound Number** | **Dose (μg/cm²)** | **1 Hour % Mortality** | **4 Hour % Mortality** |
| 9 | 2.360 | 15 | 80 |
| | 0.790 | 20 | 65 |
| | 0.079 | 0 | 50 |
| 10 | 2.360 | 5 | 95 |
| | 0.790 | 0 | 50 |
| | 0.079 | 5 | 45 |

The invention is based upon the first therapeutic method of treatment of animals and humans by means of the compounds having structural formula I. Accordingly the invention provides a compound having the structural formula I for use as a therapeutic agent for the treatment of a human or an animal.

## Claims

1. A method of treatment for controlling, preventing and protecting animals and humans from infestation and infection by arthropod parasites which comprises administering or applying to the animals, humans or their environment a parasiticidally effective amount of a compound having the structural formula

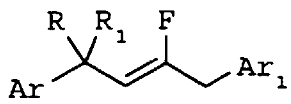

wherein

R is hydrogen or $C_1$-$C_4$alkyl, and
$R_1$ is $C_1$-$C_4$alkyl, $C_1$-$C_4$haloalkyl or cyclopropyl, or R and $R_1$ are taken together with the carbon atom to which they are attached to form a cyclopropyl group;
Ar is phenyl optionally substituted with up to three groups independently selected from halogen atoms, $C_1$-$C_4$alkyl groups, $C_1$-$C_4$haloalkyl groups, $C_1$-$C_4$alkoxy groups or $C_1$-$C_4$haloalkoxy groups, or 1- or 2-naphthyl optionally substituted with up to three groups independently selected from halogen atoms, $C_1$-$C_4$alkyl groups, $C_1$-$C_4$haloalkyl groups, $C_1$-$C_4$alkoxy groups or $C_1$-$C_4$haloalkoxy groups; and
$Ar_1$ is phenoxyphenyl optionally substituted with up to five groups independently selected from halogen atoms, $C_1$-$C_4$alkyl groups, $C_1$-$C_4$haloalkyl groups, $C_1$-$C_4$alkoxy groups or $C_1$-$C_4$haloalkoxy groups,
phenyl optionally substituted with up to five groups independently selected from halogen atoms, $C_1$-$C_4$alkyl groups, $C_1$-$C_4$haloalkyl groups, $C_1$-$C_4$alkoxy groups or $C_1$-$C_4$haloalkoxy groups,
biphenyl optionally substituted with up to five groups independently selected from halogen atoms, $C_1$-$C_4$alkyl groups, $C_1$-$C_4$haloalkyl groups, $C_1$-$C_4$alkoxy groups or $C_1$-$C_4$haloalkoxy groups,
benzylphenyl optionally substituted with up to five groups independently selected from halogen atoms, $C_1$-$C_4$alkyl groups, $C_1$-$C_4$haloalkyl groups, $C_1$-$C_4$alkoxy groups or $C_1$-$C_4$haloalkoxy groups, or
benzoylphenyl optionally substituted with up to five groups independently selected from halogen atoms, $C_1$-$C_4$alkyl groups, $C_1$-$C_4$haloalkyl groups, $C_1$-$C_4$alkoxy groups or $C_1$-$C_4$haloalkoxy groups;

and the optical isomers thereof, and the configuration of the groups $ArCRR_1$- and -$CH_2Ar_1$ about the double bond is predominantly mutually trans.

2. The method according to claim 1 wherein

R is hydrogen and $R_1$ is isopropyl, trifluoromethyl or cyclopropyl, or R and $R_1$ are methyl, or R and $R_1$ are taken together with the carbon atom to which they are attached to form a cyclopropyl group;
Ar is phenyl optionally substituted with up to three groups independently selected from halogen atoms, $C_1$-$C_4$alkyl groups, $C_1$-$C_4$haloalkyl groups, $C_1$-$C_4$alkoxy groups or $C_1$-$C_4$haloalkoxy groups; and
$Ar_1$ is 3-phenoxyphenyl optionally substituted with up to five groups independently selected from halogen atoms, $C_1$-$C_4$alkyl groups, $C_1$-$C_4$haloalkyl groups, $C_1$-$C_4$alkoxy groups or $C_1$-$C_4$haloalkoxy groups,
3-biphenyl optionally substituted with up to five groups independently selected from halogen atoms, $C_1$-$C_4$alkyl groups, $C_1$-$C_4$haloalkyl groups, $C_1$-$C_4$alkoxy groups or $C_1$-$C_4$haloalkoxy groups,
3-benzylphenyl optionally substituted with up to five groups independently selected from halogen atoms, $C_1$-$C_4$alkyl groups, $C_1$-$C_4$haloalkyl groups, $C_1$-$C_4$alkoxy groups or $C_1$-$C_4$haloalkoxy groups, or
3-benzoylphenyl optionally substituted with up to five groups independently selected from halogen atoms, $C_1$-$C_4$alkyl groups, $C_1$-$C_4$haloalkyl groups, $C_1$-$C_4$alkoxy groups or $C_1$-$C_4$haloalkoxy groups.

3. The method according to claim 2 wherein

$Ar_1$ is 3-phenoxyphenyl optionally substituted with up to five groups independently selected from halogen atoms, $C_1$-$C_4$alkyl groups, $C_1$-$C_4$haloalkyl groups, $C_1$-$C_4$alkoxy groups or $C_1$-$C_4$haloalkoxy groups.

4. The method according to claim 3 wherein

R is hydrogen and $R_1$ is isopropyl, trifluoromethyl or cyclopropyl, or R and $R_1$ are taken together with the carbon atom to which they are attached to form a cyclopropyl group;
Ar is 4-chlorophenyl, 4-fluorophenyl, 4-(trifluoromethoxy)phenyl or 4-ethoxyphenyl; and
$Ar_1$ is 4-fluoro-3-Phenoxyphenyl, *m*-(*p*-fluorophenoxy)phenyl, 4-fluoro-3-(*p*-fluorophenoxy)phenyl or 3-phenoxyphenyl.

**5.** The method according to claim 4 wherein the compound is selected from the group consisting of

1-(*p*-chlorophenyl)-1-[2-fluoro-3-(4-fluoro-3-phenoxyphenyl)propenyl]cyclopropane, (Z) -;
2,5,5,5-tetrafluoro-4-(*p*-fluorophenyl)-1-(*m*-phenoxyphenyl)-2-pentene, (Z) -;
4-(*p*-chlorophenyl)-2-fluoro-1-(4-fluoro-3-phenoxyphenyl)-5-methyl-2-hexene, (Z)-;
1-[1-(*p*-chlorophenyl)-3-fluoro-4-(4-fluoro-3-phenoxyphenyl)-2-butenyl]cyclopropane, (Z) -;
1-{1-(*p*-chlorophenyl)-3-fluoro-4-[4-fluoro-3-(*p*-fluorophenoxy)phenyl]-2-butenyl}cyclopropane, (Z) -; and
1-[3-fluoro-4-(4-fluoro-3-phenoxyphenyl)-1-[*p*-(trifluoromethoxy)phenyl]-2-butenyl)cyclopropane, (Z)-.

**6.** The method according to claim 1 which further comprises topically applying a composition containing the compound to the skin, hide, fur, feathers or hair of the animals or humans.

**7.** The method according to claim 6 wherein the animals are selected from the group consisting of cattle, sheep, horses, deer, camels, swine, goats, ferrets, mink, rabbits, amphibians, reptiles, fish, birds, poultry, dogs and cats.

**8.** The method according to claim 6 for controlling and preventing infestation and infection by flies, fleas, lice, mosquitoes, gnats, mites and ticks.

**9.** The method according to claim 1 wherein the compound is orally, intragastrically or intraruminally administered to the animals or humans.

**10.** The method according to claim 1 wherein the compound is parenterally administered to the animals or humans.

**11.** The method according to claim 1 wherein the animals are selected from the group consisting of cattle, sheep, horses, deer, camels, swine, goats, ferrets, mink, rabbits, amphibians, reptiles, fish, birds, poultry, dogs and cats.

**12.** The method according to claim 1 wherein the compound is administered or applied in combination with or in conjunction with one or more synergists.

**13.** The method according to claim 1 wherein the compound is administered or applied in combination with or in conjunction with one or more other parasiticidal compounds.

**14.** The method according to claim 1 wherein a composition containing the compound is applied to the animal's or human's environment.

**15.** A composition for the treatment of an animal or human by therapy which composition comprises an veterinarily or pharmaceutically acceptable carrier and a parasiticidally effective amount of a compound having the structural formula

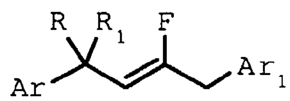

wherein R, $R_1$, Ar, and $Ar_1$ are defined as in claim 1.

**16.** The composition according to claim 17 wherein R,$R_1$, Ar, and $Ar_1$ are as defined in claim 2.

**17.** The composition according to claim 16 wherein

$Ar_1$ is 3-phenoxyphenyl optionally substituted with up to five groups independently selected from halogen atoms, $C_1$-$C_4$alkyl groups, $C_1$-$C_4$haloalkyl groups, $C_1$-$C_4$alkoxy groups or $C_1$-$C_4$haloalkoxy groups.

**18.** The composition according to claim 17 wherein

R is hydrogen and $R_1$ is isopropyl, trifluoromethyl or cyclopropyl, or R and $R_1$ are taken together with the carbon atom to which they are attached to form a cyclopropyl group;
Ar is 4-chlorophenyl, 4-fluorophenyl, 4-(trifluoromethoxy)phenyl or 4-ethoxyphenyl; and
$Ar_1$ is 4-fluoro-3-phenoxyphenyl, *m*-(*p*-fluorophenoxy)phenyl, 4-fluoro-3-(*p*-fluorophenoxy)phenyl or 3-phenoxyphenyl.

**19.** The composition according to claim 15 which further comprises one or more synergists.

**20.** The composition according to claim 15 which further comprises one or more other parasiticidal compounds.

**21.** A compound for the therapeutic treatment of an animal or human, the compound being chosen from those having the structural formula

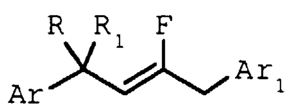

wherein Ar, R, $R_1$ and $Ar_1$ are as defined in claim 1 and the optical isomers thereof, wherein the configuration of the groups $ArCRR_1$- and $-CH_2Ar_1$ about the double bond is predominantly mutually trans.

European Patent Office

# EUROPEAN SEARCH REPORT

Application Number
EP 99 30 0561

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| X,D | GB 2 288 803 A (BRITISH TECH GROUP) 1 November 1995<br>* claims *<br>* page 4, line 1 - line 2 * | 1-21 | A01N31/04 |
| X | BEDDIE D G ET AL: "THE PYRETHRIN AND RELATED COMPOUNDS. PART XLI. STRUCTURE-ACTIVITY RELATIONSHIPS IN NON-ESTER PYRETHROIDS AGAINST RESISTANT STRAINS OFHOUSEFLY (MUSCA DOMESTICA L.)" PESTICIDE SCIENCE, vol. 48, no. 2, 1 October 1996, pages 175-178, XP000682558<br>* table 1 * | 1-21 | |
| A,D | WO 97 16067 A (BRITISH TECH GROUP ;KHAMBAY BHUPINDER PALL SINGH (GB); CAHILL MATT) 9 May 1997 | | |
| A,D | WO 94 06741 A (LIU MU GUANG ;BRITISH TECH GROUP (GB); KHAMBAY BHUPINDER PALL SING) 31 March 1994 | | TECHNICAL FIELDS SEARCHED (Int.Cl.6)<br>A01N |

The present search report has been drawn up for all claims

| Place of search | Date of completion of the search | Examiner |
|---|---|---|
| THE HAGUE | 26 May 1999 | Decorte, D |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 99 30 0561

This annex lists the patent family members relating to the patent documents cited in the above-mentioned European search report.
The members are as contained in the European Patent Office EDP file on
The European Patent Office is in no way liable for these particulars which are merely given for the purpose of information.

26-05-1999

| Patent document cited in search report | Publication date | Patent family member(s) | Publication date |
|---|---|---|---|
| GB 2288803 A | 01-11-1995 | AU 2264995 A | 29-11-1995 |
| | | BG 100934 A | 30-12-1997 |
| | | BR 9507508 A | 02-09-1997 |
| | | CA 2187918 A | 09-11-1995 |
| | | CN 1147242 A | 09-04-1997 |
| | | CZ 9603107 A | 13-08-1997 |
| | | EP 0757668 A | 12-02-1997 |
| | | FI 964343 A | 28-10-1996 |
| | | WO 9529887 A | 09-11-1995 |
| | | GB 2301360 A | 04-12-1996 |
| | | HU 76430 A,B | 28-08-1997 |
| | | JP 9512543 T | 16-12-1997 |
| | | NO 964563 A | 28-10-1996 |
| | | PL 317027 A | 03-03-1997 |
| | | SK 128996 A | 04-06-1997 |
| | | US 5880162 A | 09-03-1999 |
| | | ZA 9503119 A | 09-01-1996 |
| WO 9716067 A | 09-05-1997 | AU 7318896 A | 22-05-1997 |
| | | EP 0876099 A | 11-11-1998 |
| WO 9406741 A | 31-03-1994 | AT 151737 T | 15-05-1997 |
| | | CA 2142714 A | 31-03-1994 |
| | | CN 1087895 A,B | 15-06-1994 |
| | | DE 69309940 D | 22-05-1997 |
| | | DE 69309940 T | 04-09-1997 |
| | | DK 660815 T | 26-05-1997 |
| | | EP 0660815 A | 05-07-1995 |
| | | ES 2101343 T | 01-07-1997 |
| | | GB 2270693 A,B | 23-03-1994 |
| | | GR 3024029 T | 31-10-1997 |
| | | HU 71731 A | 29-01-1996 |
| | | JP 8504186 T | 07-05-1996 |
| | | US 5763700 A | 09-06-1998 |
| | | ZA 9306834 A | 16-03-1995 |

EPO FORM P0459

For more details about this annex : see Official Journal of the European Patent Office, No. 12/82